# EUROPEAN PATENT APPLICATION

(11) **EP 2 502 633 A1**
(43) Date of publication of application: **26.09.2012**
(21) Application number: 12156526.1
(22) Date of filing: 22.02.2012
(51) Int. Cl.: A61K 38/28, C07K 14/62, C12P 21/02

(54) **Recombinant plasmid DNA pMSIN4, encoding a hybrid polypeptide comprising human insulin precursor, E. coli strain BL21 (DE3) / pMSIN4 - producer of recombinant human insulin, the method for the recombinant human insulin production**

(30) Priority: 24.03.2011 RU 2011111205
(71) Applicant: Winsort Management Inc., Belize City (BZ)
(72) Inventor:
(74) Representative: Zaccaro, Elisabetta

(57) **Abstract**

The invention refers to biotechnology, particularly genetic engineering, and can be used in the production of recombinant human insulin.

The technical result, at which the invention is aimed, is to increase the quality and quantity of yield of the desired product - recombinant human insulin, as well as to simplify the industrial production of recombinant human insulin.

The result is achieved by constructing a recombinant plasmid pMSIN4, encoding a hybrid polypeptide - a precursor of human insulin, a strain VL21 (DE3) / pMSIN4 - producer of recombinant human insulin, as well as by optimizing the way of the industrial production of recombinant human insulin.

The inventions therefore allows to simplify the process of the industrial production of the high purity recombinant human insulin, as well as to increase the yield of the desired product.

## Description

### Field of the invention

The invention is related to the field of biotechnology, in particular to genetic engineering, and can be used in the production of recombinant human insulin.

### State of the art

Insulin is a protein (peptide) hormone produced in animals and humans by the cells of the pancreas Langerhans islets in the form of its predecessor - preproinsulin, which consists of 109 amino acid residues. In the process of biosynthesis, as a result of the 23 amino acid signal peptide cleavage, proinsulin is released, which is then subjected to processing, leading to the formation of proper insulin due to the specific "excision" of the 35 amino acid C-peptide. Biologically active mature insulin consists of two chains connected by two disulfide bonds. The A chain contains 21 and the B chain - 30 amino acid residues.

Insulin lowers blood sugar by delaying the breakdown of glycogen in the liver and increasing the use of glucose by the muscle and other cells. Lack of insulin leads to the development of such diseases as diabetes.

This disease is treated by compensating the lack of this hormone with the help of medical preparations containing monocomponent insulin. Earlier in the medical practice, the treatment of insulin-dependent diabetes (Type 1) made extensive use of the insulin of animal origin, obtained by the extraction from the pancreas of pigs or cattle. However, the use of these drugs often leads to complications caused by the potential immunogenicity of foreign proteins.

The need for insulin word-wide is estimated to be 18,000.00 kg/year.

Currently, a promising method for obtaining human insulin is a method based on the technology of recombinant proteins. The most common way of obtaining the desired product is that of microbiological synthesis by the introduction of the gene responsible for synthesis of a substance into the recipient strain cell. As a rule, a vector is used to introduce a gene into the recipient strain cell. The vector in particular contains the expression cassette (promoter, transcription terminator, selectable marker). The synthesis of the desired product is achieved by culturing the recombinant producer strain.

Methods for producing recombinant human insulin are known in the state of the art, including the cultivation of producer strains expressing the precursor protein, the refolding of the protein precursor, its proteolytic conversion and subsequent purification steps of the insulin obtained. Among the many sources of information on obtaining a recombinant insulin, there are studies that describe the genetic constructs encoding the precursor protein, differing in molecular weight within the range from 16.7 to 47 kDa due to the different amino acid sequences of the leader design prepeptide and C-peptide - the portion, binding amino acid sequences of the A- and B-chain.

As leader sequences protecting the recombinant protein from the proteolytic degradation a sequence of glutathione S-transferase is used, for example. It is known that in the absence of the leader prepeptide, the synthesis of the insulin precursor in *E. coli* cells is inhibited by the high cytotoxicity of the proinsulin sequence. As a leader sequences protecting the recombinant protein from proteolytic degradation, for example, the following sequences have been used: glutathione S-transferase (see Berg H., Walter M., Mauch L., Seissler J., Northemann W. Recombinant human preproinsulin expression, purification and reaction with insulin autoantibodies in sera from patients with insulin-dependent diabetes mellitus. 1993, Journal of Immunological Methods, v. 164, p. 221-231), IgG-binding domain (see Jonasson P., Nilsson J., Samuelsson E ., Moks T., Stahl S., Uhlen M. Single-step trypsin cleavage of a fusion protein to obtain human insulin and its C peptide. 1996, European Journal of Biochemistry, v. 236, p. 656-661), the sequence containing six histidine residues (see Berg H., Walter M., Mauch L., Seissler J., Northemann W. Recombinant human preproinsulin expression, purification and reaction with insulin autoantibodies in sera from patients with insulin-dependent diabetes mellitus. 1993 Journal of Immunological Methods, v. 164, p. 221-231), maltose-binding domain, beta-galactosidase (Cho Sh.W., Park SH, Nam DH Production and purification of single chain human insulin precursors with various fusion peptides. 2001, Biotechnology and Bioprocess Engineering, v. 6, p. 144-149) etc.

The leader fragment design is carried out taking into account the fact that the human proinsulin, synthesized in the fusion protein, is accumulated in bacterial cell as insoluble conglomerates, the so-called "inclusion bodies". In addition, the leader fragment is required for the initiation of translation through the starting methionine content.

Obviously, the larger the insulin precursor molecule, the lower the yield of the final product. This is due to the decrease in the percentage of the amino acid sequence of insulin to the total amino acid content of its precursor.

From the state of the art we know the method of producing the recombinant human insulin, which consists in the cultivation of producer strain *E. coli* producing the hybrid protein which, besides the proinsulin, contains two synthetic IgG-binding domains of staphylococcal protein A. The insulin release scheme involves: the destruction of the bacterial cells to obtain the inclusion bodies containing the fusion protein, the dissolution of the inclusion bodies, the oxidative sulfitolysis of proinsulin, its renaturation, the renatured protein treatment by affinity chromatography on IgG-Sepharosa, the proinsulin cleavage by proteolytic enzymes (trypsin and carboxypeptidase B) and the final purification of insulin by the reversed-phase high performance liquid chromatography (see Nilson J., Jonasson P., Samuelsson E., Stahi S.. Uhlen M., «Integrated production of human insulin and its C-peptide», Jurnal of bioteshnology, 1996, v. 48, p. 241-250).

This method has the disadvantages of being very expensive. In fact such a method requires the use of a rich nutrient medium for growing the strain-producer and requires the use of and expensive affinity sorbent for cleaning. Furthermore the desired product has a short shelf-life and therefore difficult to be applied industrially.

In addition, this method also has the disadvantage of a low yield of the desired product.

The following method for producing the genetically engineered human insulin is known from Russian Patent # 2208637 "Method of production of genetically engineered human insulin", filing date 16.04.2002, published on 20.07.2003. This method comprises the steps of: cultivation of the producer strain *Escherichia coli* JM 109pPINS07, destruction of the cells by disintegration, separation of the inclusion bodies containing the fusion protein, and their dissolution in a buffer containing urea and dithiothreitol, restoration of the disulfide bonds, refolding and purification of the renatured hybrid protein, its cleavage by trypsin and carboxypeptidase B, followed by the purification and separation of insulin, before the preliminary washing of the inclusion bodies is carried out, and the dissolution of the protein and the restoration of disulfide bonds is carried out simultaneously in a buffer solution, additionally containing 5-10 mm of dithiothreitol, for 3-7 hours, the cleaning of the renatured fusion protein is carried out in one step using ion-exchange sorbents with DEAE- or SP-groups, the fusion protein cleavage is carried out by the co-hydrolysis with trypsin and carboxypeptidase B at a weight ratio of the fusion protein, trypsin and carboxypeptidase B 4000:1-2:1, the insulin treatment is carried out using the hydrophobic chromatography sorbent Butyl-Toyopearl 650, followed by gel-filtration, and separation - using the crystallization in the presence of zinc salts (see Russian Patent For Invention # 2208637 "Method of production of genetically engineered human insulin", filing date 16.04.2002 , published on 20.07.2003).

Russian Patent # 2144957 "Recombinant plasmid DNA rRINS07 encoding a hybrid polypeptide comprising proinsulin man, and a strain of *Escherichia coli -* a producer of hybrid polypeptide comprising proinsulin man," the filing date 26.02.1999, published on 27.01.2000 describes the recombinant plasmid DNA rRINS07 encoding the hybrid polypeptide with the sequence of human proinsulin. In this plasmid the sequence of domain B of staphylococcal protein A is connected via a peptide linker His6GIySerArg with the amino acid sequence of human proinsulin, with a molecular mass of 3.3 MDa (5051 bp) containing the EcoRl /Hindlll-fragment of plasmid rKK223-3, including the hybrid tac-promoter, β-lactamase gene (bla), the replication initiation section (ori) and the transcription terminator of the ribosomal operon *E. coli,* EcoRl / BamHl-fragment (220 bp) with an artificial gene encoding the IgG-binding domain of protein A from Staphylococcus aureus and hexahistidine domain, and the BamHl / Hindlll-fragment (271 bp) encoding the human arginilproinsulina sequence, the genetic marker - a gene β-lactamase (bla), which determines the resistance of the cells, transformed with plasmid rRINS07 *E. coli,* to ampicillin, E.coli, a unique restriction endonuclease recognition sites at these coordinates: EsoRl-1, Ncol - 217, BamHl - 221, Pstl - 342 and 417, Hindlll - 492, Sall - 4513, Clal - 4792.

The closest prior art is the method described in Russian Patent # 2141531 "Method for production of recombinant human insulin," filing date 26.05.1999, published on 20.11.1999. It describes a method for obtaining the recombinant human insulin, including the steps of cultivation of producer strain *Escherichia coli,* the disruption of cells by disintegration, the separation of inclusion bodies containing the fusion protein, and their dissolution in a buffer containing urea and dithiothreitol, the renaturation and purification of re-natured fusion protein and its cleavage by trypsin and carboxypeptidase B followed by the purification and obtaining of the desired product, wherein *Escherichia coli* JM1 09/pPINS07 is used as a producer strain. The re-natured fusion protein purification is carried out by the precipitation of impurity compounds in 40% isopropanol followed by chromatography on CM-Sepharose, the fusion protein cleavage by trypsin and carboxypeptidase B is carried out consistently, whereas the tripsinolysis products are chromatographed on SP-Sepharose equilibrated with 0.003 - 0.1 M ammonium acetate buffer, pH 5.0-6.0, containing 6M urea, with the fraction elution with a linear gradient sodium chloride, 0 - 0.5 M in the starting buffer, and the insulin fraction obtained after cleavage by carboxypeptidase B was purified by HPLC on reverse phases, followed by the gel filtration.

Therefore, it is the object of the present invention to identify an improved recombinant human insulin product and a method which allows to increase the quality and quantity of yield of the desired product: recombinant human insulin, as well as to simplify it's industrial production.

### Summary of the invention

The present invention provides an *E.coli* strain VL21 (DE3) (VL21 (DE3) /pMSIN4) which is deposited under deposit Accession No. SMS 7884, with International Deposit Authority Czech Collection of Microorganisms, Masaryk University, Brno, Czech Republic.

A further embodiment of the invention regards a recombinant human insulin produced by the *E.coli* strain VL21 (DE3) (VL21 (DE3)/pMSIN4) which is deposited under deposit Accession No. SMS 7884, with International Deposit Authority Czech Collection of Microorganisms, Masaryk University, Brno, Czech Republic, or fragments and derivatives thereof.

A further embodiment of the invention regards a recombinant human insulin of the present invention, for use as a medicament.

A still further embodiment of the invention regards a recombinant human insulin of the present invention, for use in the therapeutic treatment of diabetes.

In a still further embodiment the invention relates to a pharmaceutical composition comprising the recombinant human insulin according to the invention as an active ingredient and a pharmaceutically bioactive excipient.

Exemplary pharmaceutically acceptable excipients include any and all solvents, dispersion media, diluents, or other liquid vehicles, dispersion or suspension aids, surface active agents, isotonic agents, thickening or emulsifying agents, preservatives, solid binders, lubricants and the like, as suited to the particular form of administration and dosage.

In a further embodiment the invention relates to a recombinant plasmid for the expression of recombinant pMSIN4 human insulin having a size of 5495 bp, wherein said recombinant plasmid consists of the following elements:
- a resistance gene to kanamycin (Kanr, 813 bp);
- a cloned gene (synthetic gene, 225 bp) encoding the insulin precursor protein, containing a prepeptide consisting of 21 amino acid residues (2.46 kDa);
- a T7 promoter (17 bp);
- an arginine codon (CGC), linking the sequences encoding the A- and B-insulin chain; and
- a replication site (ColE1 origin, 588 bp).

In a further embodiment the invention relates to the use of the recombinant pMSIN4 plasmid for the expression of recombinant human insulin.

In a still further embodiment the invention relates to a method for the production of recombinant human insulin, comprising the steps of:
a. culturing of the producer strain *Escherichia coli* according to claim 1 in a nutrient medium; and
g. purifying and obtaining the recombinant human insulin.

### Brief description of the drawings

The characteristics and advantages of the present invention will be apparent from the detailed description reported below, from the Examples given for illustrative and non-limiting purposes, and from the annexed Figure, wherein:

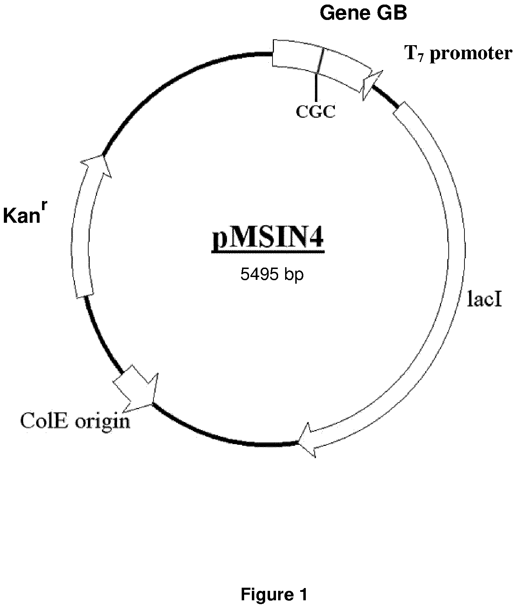

Figure 1 shows a physical map of plasmid rMSIN4 where
Kanr - kanamycin resistance gene (813 bp);
Gene GB - cloned gene (225 bp) encoding the insulin precursor protein and containing prepeptide, consisting of 21 amino acid residues (2.46 kDa);
T7 promoter - T7 promoter (17 bp);
ori - a replication site (ColE1 origin, 588 bp).

### Detailed description of the invention

The present invention provides an *E.coli* strain VL21 (DE3) (VL21 (DE3) /pMSIN4) which is deposited under deposit Accession No. SMS 7884, with International Deposit Authority Czech Collection of Microorganisms, Masaryk University, Brno, Czech Republic.

A further embodiment of the invention regards a recombinant human insulin produced by the *E.coli* strain VL21 (DE3) (VL21 (DE3)/pMSIN4) which is deposited under deposit Accession No. SMS 7884, with International Deposit Authority Czech Collection of Microorganisms, Masaryk University, Brno, Czech Republic, or fragments and derivatives thereof.

The recombinant human insulin produced by the *E.coli* strain VL21 (DE3) (VL21 (DE3)/pMSIN4) according to the present invention provides the advantages of being of a higher quality of insulin and of having a degree of purification with a purity of not less than 96%. Furthermore the recombinant human insulin according to the present invention has an activity of over 26 U/mg.

A further embodiment of the invention regards a recombinant human insulin of the present invention, for use as a medicament.

A still further embodiment of the invention regards a recombinant human insulin of the present invention, for use in the therapeutic treatment of diabetes.

In a further embodiment the invention relates to the use of the recombinant human insulin of the present invention, wherein said diabetes is type I or II diabetes.

In a still further embodiment the invention relates to a pharmaceutical composition comprising the recombinant human insulin according to the invention as an active ingredient and a pharmaceutically bioactive excipient.

In a further embodiment the invention relates to a recombinant plasmid for the expression of recombinant pMSIN4 human insulin having a size of 5495 bp, wherein said recombinant plasmid consists of the following elements:
- a resistance gene to kanamycin (Kanr, 813 bp);
- a cloned gene (synthetic gene, 225 bp) encoding the insulin precursor protein, containing a prepeptide consisting of 21 amino acid residues (2.46 kDa);
- a T7 promoter (17 bp);
- an arginine codon (CGC), linking the sequences encoding the A- and B-insulin chain; and
- a replication site (ColE1 origin, 588 bp).

The claimed recombinant plasmid for the expression of recombinant pMSIN4 human insulin, which has a size of 5495 bp, consists of the following elements:
resistance gene to kanamycin (Kanr, 813 bp), a cloned gene (225 bp) encoding the insulin precursor protein, containing a prepeptide, consisting of 21 amino acid residues (2.46 kDa), a T7 promoter (17 bp), an arginine codon (CGC), linking the sequences encoding the A- and B-chains of insulin, and a replication section (ColE1 origin, 588 bp).

The peculiarity of the above-mentioned plasmid is that it contains a synthetic gene encoding a protein - the precursor of insulin, containing small-size prepeptide, consisting of 21 amino acid residues (2.46 kDa). The structure of the gene encoding proinsulin section, differs from the corresponding native gene human proinsulin section, namely, the section encoding the C-peptide is removed from the DNA sequence, and the codon of arginine, which binds the sequence encoding the A- and B-chain of insulin, is introduced instead.

This difference provides a higher yield of the desired product by increasing the percentage of insulin from the proinsulin part, as confirmed by the structures of native (A) and synthetic proinsulin (B) given below.

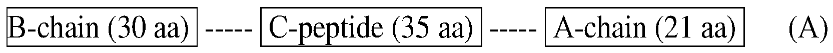

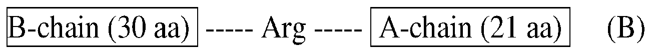

Thus, native insulin contains 86 amino acid residues, of which insulin consists of 51 amino acid residues. Thus, insulin in the native part of the proinsulin equals 59.3%. In the proposed construct the proinsulin part contains 52 amino acid residues, and consequently, the insulin accounts for 98% of the proinsulin part or 68.9% of the fusion protein. For improved expression of the fusion protein and increase in the yield of the synthesized product, the cloned sequence is optimized for the triplets that are characteristic of tRNA *E.coli.*

The established plasmid DNA was introduced into *Escherichia coli* cells (*E.coli*) strain BL21 (DE3).

The strain VL21 (DE3) / pMSIN4 (deposited in the Czech Collection of Microorganisms (Czech Collection of Microorganisms, Masaryk University, Brno, Czech Republic, identification number CCM 7884) was obtained by transforming *E.coli* cells strain BL21 (DE3) with the recombinant plasmid pMSIN4.

When culturing the producer strain BL21 (DE3) / pMSIN4 the bacterial culture is grown to 100-120 optical units, thus increasing the yield of inclusion bodies compared with the prototype.

In a preferred embodiment the recombinant plasmid according to the invention, has a resistance gene which is a resistance to kanamycin (Kanr, 813 bp).

In a further embodiment the invention relates to the use of the recombinant pMSIN4 plasmid for the expression of recombinant human insulin.

In a still further embodiment the invention relates to a method for the production of recombinant human insulin, comprising the steps of:
a. culturing of the producer strain *Escherichia coli* according to claim 1 in a nutrient medium; and
h. purifying and obtaining the recombinant human insulin.

The method according to the present invention provides the advantages of achieving a higher quality of insulin and a degree of purification with a purity of not less than 96%. Furthermore the method allows to achieve the production of recombinant human insulin having an activity of over 26 U/mg.

The technical result, which the method of the invention achieves, is that of increasing the quality and quantity of yield of the desired product -the recombinant human insulin, as well as simplifying the industrial production of such recombinant human insulin.

In a preferred embodiment the method comprises the steps of:
a. culturing of the producer strain *Escherichia coli* according to claim 1 in a nutrient medium,
b. destroying the cells by disintegration,
c. separating the inclusion bodies containing the fusion protein,
d. dissolving the inclusion bodies in the buffer with chaotropic agents,
e. allowing fusion protein refolding,
f. cleaving the protein with trypsin and/or carboxypeptidase B
g. purifying and obtaining the recombinant human insulin.

In a still preferred embodiment the invention relates to a method, wherein step b. is carried out using a homogenizer, and wherein after step e. the protein is concentrated by depositing it in the isoelectric point in the presence of zinc ions. In this further preferred method the trypsin cleavage of step f. is carried out at a weight ratio of 5000-20000:1 and at a pH of 11.0-11.5, after which a phased cleanup is performed. The phased cleanup includes the cleanup on reversed-phase sorbents, including using a polymeric sorbent or modified silica gel, eluting with a linear gradient to produce a concentration of 2-propanol, and ion-exchange chromatographic purification on polymeric sorbents, followed by carboxypeptidase B cleavage carried out at a weight ratio of 10000-50000:1 and a final cleaning using the modified reversed-phase silica gel, with elution of the fractions by the binary linear gradient for the concentration of 2-propanol.

In a still preferred embodiment the invention relates to a method, wherein as modified spherical sorbent silica gel, Daisogel SP-120-10-C8-Bio, as well as the sorbent Amberchrom XT-20 as a polymeric sorbent is used.

In detail, the claimed method for the industrial production of recombinant human insulin includins the cultivation of producer strain *Escherichia coli* in a nutrient medium, cell disruption by disintegration, separation of inclusion bodies containing the fusion protein, and their dissolution in a buffer with chaotropic agents, refolding of the fusion protein, its gradual splitting with trypsin and carboxypeptidase B, followed by the purification to obtain the desired product, uses, as a producer strain, according to the invention, the strain VL21 (DE3) / pMSIN4, carrying the recombinant plasmid pMSIN4; the cell disruption is carried out using a homogenizer, after refolding the fusion protein concentration is carried out by depositing it in the isoelectric point in the presence of zinc ions, the trypsin-triggered cleavage is carried out at a weight ratio 5000-20000:1 and pH 11,0-11,5, after which a phased cleanup is conducted, including the cleanup on reversed-phase sorbents, in particular, using the polymeric sorbent or modified silica gel, eluting with a linear gradient to produce a concentration of 2-propanol, and ion-exchange chromatographic purification on polymeric sorbents, followed by the carboxypeptidase B cleavage, carried out at a weight ratio 10000-50000:1, and the final cleaning using a modified reversed-phase silica gel with the fractions elution by the binary linear gradient of 2-propanol concentration.

As a homogenizer the homogenizer «Microfluidizer» is used.

As a modified spherical sorbent, silica gel Daisogel SP-120-10-C8-Bio is used, and as polymeric sorbent the sorbent Amberchrom XT-20 is used.

The prior art methods do not provide a detailed description of the process of obtaining inclusion bodies, or the description refers to a laboratory, and, hence, unproductive method in which the destruction of cells is carried out by the ultrasonic influence or by treatment with lysozyme. The use of ultrasound on the industrial scale is inefficient because of the low rate of bacterial cells destruction (50-60%), and the use of lysozyme leads to the appreciation of the final product due to the high cost of this enzyme.

Refolding involves the following steps: the dissolution of inclusion bodies in the presence of chaotropic agents, restoration of disulfide bonds, the protein renaturation with the formation of native conformation and the concentration of the refolded protein.

The two alternative ways for the insulin precursor renaturation are described in the literature: the one is based on the use of oxidative sulfitolysis of the cysteine residues SH-groups (see Zhigis LS, Rapoport EM, Zueva VS, Reshetov PD A study of human recombinant insulin S-sulfonation conditions. 1993 , Pharmaceutical Chemistry Journal, v. 27, p. 789-796.); the second is based on the formation of disulfide bonds through the oxidation of reduced SH-groups in the medium with the appropriate redox potential. The second way of the insulin precursor renaturation is superior due to reducing the number of process steps and reagents used.

The insulin precursor renatured protein concentration stage suggested by the present invention is inexpensive and takes much less time than the approaches described in the prior art. Typically, the concentration of the refolded insulin precursor is performed using the chromatographic techniques or by ultrafiltration. On the industrial scale both of these approaches require the use of costly basic equipment and consumables, such as sorbents for chromatography and membrane cassettes for tangential ultrafiltration.

For the proteolytic conversion of the insulin precursor multiple stages and a diverse set of enzymes are used. Thus, the removal of leader fragment requires cyanogen bromide, thrombin, factor Xa, etc., as well as trypsin, depending on the design and the availability of appropriate prepeptide site for the proteolytic enzyme. The use of cyanogen bromide is limited because of its high toxicity, and the use of specific proteases such as thrombin, factor Xa, etc., is not cost-efficient at large production volumes due to the high cost of the latter.

The presence of the C-terminal Arg in the sequence of leader fragment is very advantageous and enables us to use trypsin for its cleavage, which is more feasible. In addition, for the cleavage of the insulin precursor proinsulin part trypsin with carboxypeptidase B is used, while the splitting is carried out either in two stages by performing the sequential processing, or by processing the both enzymes simultaneously. The joint processing with these enzymes reduces the amount of enzyme steps in the process, but makes it more difficult to control the tripsinolysis, as, besides insulin, other proteolytic products are inevitably released during the treatment, namely, Des-(B30) Thr-insulin and Des-octapeptide (B23-B30)-insulin . In addition, the co-processing with trypsin and carboxypeptidase B significantly increases the consumption of carboxypeptidase B (by 10 to 15).

The claimed technical solution offers an optimized proteolytic conversion scheme of the insulin precursor protein with a specific output of insulin not less than 60% of the refolded fusion protein weight, and the technology to purify the intermediate Arg-(B31)-insulin and insulin, which eliminates the above-mentioned disadvantages. This scheme does not provide for the purification of hybrid protein, which eliminates the need for large amounts of urea and ion-exchange sorbents, which, in turn, both reduces the cost of technology and simplifies it.

A number of works suggested many schemes to clean and get highly purified insulin, including, in particular, affinity chromatography, gel permeation chromatography, hydrophobic interaction chromatography, ion exchange chromatography and HPLC on a drawn-phase sorbents. But the established schemes have drawbacks, in particular, due to the high cost of the chromatographic process because of the use of affine sorbents, large consumption of urea and sorbents used for the purification of the fusion protein, as well as multistage, low yields of the desired product, poor selectivity, etc. Technical solutions that match the set of essential features of the claimed invention have not been revealed, which allows us to conclude that the claimed invention meets such a condition of patentability as "novelty".

The claimed essential features that predetermine achieving the said technical result, do not obviously stem from the prior art, which allows us to conclude that the claimed invention meets such a condition of patentability as "inventive step". The patentability condition of "industrial applicability" is confirmed by the application examples.

The implementation of the proposed method of producing the recombinant human insulin is confirmed and illustrated in the Examples 1 - 9 of specific performance described in the present invention.

### EXAMPLE 1

### Creating a plasmid pMSIN4

The synthetic gene encoding the insulin precursor is produced by the assembly in the polymerase chain reaction (PCR) using oligonucleotide primers # 1 - # 3, given below # 1. 
AAAGAAGCCACGCTCGCCGCACACTAAATACAGCGCTTCCACCAGGTGGCT
GCCACACAGATGCTGGTTCACAAAGCGCATATGAG (SEQ ID NO:1);
 # 2. 
TGCGGCGAGCGTGGCTTCTTTATACCCCGAAAACCCGTGGCATTGTGGAACA
G
 TGTTGCACCAGTATTTGTAGCCTGTATC (SEQ ID NO:2); # 3.

CATTGAATTCTTAGTTGCAGTAATTTTCCAGCTGATACAGGCTACAAATACT
GGTGCAAC (SEQ ID NO:3).

To amplify the PCR-product primers # 4 and # 5 are used.
# 4. CTCATATGCGCTTTGTGAACCAGCAT (SEQ ID NO:4);
# 5. CATTGAATTCTTAGTTGCAGTAATT (SEQ ID NO:S).

The assembly and gene amplification is carried out in a single PCR reaction, for which the reaction mixture is fed with 5 pM primers # 1 - # 3 (SEQ ID NO:1, 2 and 3) and 50 nM primers # 4 and # 5 (SEQ ID NO:4 and 5). The PCR reaction is carried out according to the protocol of the manufacturer of the PCR-kit (Fermentas ## EP0501), in TABLE 1::

**TABLE 1**

| | |
|---|---|
| 95°C - 45 sec, | 1 cycle |
| 55°C - 60 sec, | |
| 72°C - 60 sec, | |
| 95°C - 45 sec, | 35 cycles |
| 62°C - 60 sec, | |
| 72°C - 30 sec, | |

Then the purified PCR product and vector DNA (rET28a) is treated for 1 hour by nucleases Ndel and EcoRl, according to the protocols (Fermentas # ER0581, # ER0271). The restriction products are purified of the agarose gel using a set of GeneClean (Qbiogene # 1001-200) and ligated under standard conditions (Fermentas # EL0011). After that 2 µl of the obtained ligase mixture is used for the transformation of *E.coli* cells strain DH5α. The selection of positive clones is carried out using 30 µg/ml of the kanamycin sulfate. From a number of individual colonies a plasmid DNA is released, which is analyzed using the restriction analysis and sequencing. The created plasmid DNA with the same sequence as calculated is used to transform *E.coli* cells strain BL21 (DE3).

### EXAMPLE 2

### Cultivation of the producer strain BL21 (DE3) / pMSIN4

The contents of the vial of the producer strain BL21 (DE3) / pMSIN4 working bank are inoculated into 2-liter flask containing 1 liter of LB medium, containing 30 µg/ml of kanamycin. Within 12 hours the flask is incubated in the thermoshaker at the speed of 250 rev/min. and a temperature of 37°C, after which the contents of the flask are fed to the inoculating apparatus, which is a fermenter with a working volume of 75 I containing culture medium, the composition of which is shown in the Table 2.
The cultivation is carried out under the automatic temperature control (37°C ± 0,5 °C), pH (7,0 ± 0,2), dissolved oxygen range (50% <DO2 <70%). The correction of the dissolved oxygen level is done by changing the mixer speed and the correction of the pH level - by adding the concentrated ammonium hydroxide. The fermentation process is performed until the culture achieves the optical density equal to OD600 = 10 ± 2 OE, after which the contents of the inoculating unit is transferred via sterile pipelines to the main fermenter with a working volume of 2,000 liters containing the culture medium, the composition of which is shown in the Table 2.

The cultivation of strain in the fermenter is carried out at the same modes as in the inoculating apparatus, the only difference being that, starting from the point of the pH active growth (usually at OD600 = 5 ± 1 OE), the fermenter is supplied in a continuous flow with the nutritious mixture containing the 30% of glucose solution and 0.5% of the magnesium sulfate solution. The glucose feed rate is adjusted automatically depending on the dissolved oxygen level (50% <DO2 <70%). The fermentation is carried out until the culture reaches the optical density of OD600 = 50±5 OE, and then the inductor IPTG is fed to a final concentration of 1 mM. The cultivation under the indicated conditions continues for 6 hours, as a result of which the bacterial culture reaches an optical density of 100-150 OE.

**TABLE 2**

| **Component** | **Concentration, (g/I)** |
|---|---|
| Bacto peptone | 5 |
| Yeast extract | 5 |
| Glucose | 2 |
| Sodium chloride | 1 |
| Ammonium chloride | 1 |
| Potassium phosphate dibasic | 2 |
| Ammonium phosphate monobasic | 2 |
| Ammonium sulfate | 1 |
| Potassium sulphate | 1 |
| Magnesium sulfate | 0.5 |
| Ferrous sulfate heptahydrate | 0.01 |
| Manganese chloride heptahydrate | 0.001 |
| Copper sulphate | 0.005 |
| Zinc sulfate | 0.01 |
| Calcium chloride | 0.01 |
| Sodium tetraborate | 0.001 |
| Sodium molybdate | 0.0005 |
| Thiamine hydrochloride | 0.005 |
| Kanamycin sulfate | 0.03 |

### EXAMPLE 3

### Obtaining the inclusion bodies

Upon completion of culturing the producer strain, the contents of the fermenter are cooled to 10°C ± 2°C and fed to the self-discharging separator with a flow rate of 1000±100 1/hour. It is established that in this mode the losses from the non-precipitated cells do not exceed 5%, whereas the reduced flow rate does not significantly affect the increase in the number of deposited cells. The concentrated bacterial mass is diluted to a density of OD₆₀₀ = 250 ± 50 OE with the cooled (+10°C±2°C) solution of the following composition: 20 mM Tris-HCl, pH 8.0; 1 mM EDTA.

The resulting suspension is twice fed through the nanohomogenizer, the function of which is performed, for example, by «Microfluidizer», which carries out cell disruption at a pressure of 15,000 psi. It was established experimentally that a single pass-through of the suspension through the nanohomogenizer under the compliance with the above conditions leads to the destruction of 80-85% of the cells, whereas the re-transmission increases the percentage of damaged cells to 98-99%.

The destroyed biomass is fed at 2000±500 I/h to the self-discharging separator. The resulting suspension is diluted to 1000 I to wash the following composition with a cooled buffer: 20 mM Tris-HCl, pH 8.0; 1 mM EDTA, 1% Triton X-100, and incubated while stirring at a speed of 100±20 rev/min. for 30 minutes, and then fed to the separator. The bodies washing cycle is repeated three times.

### EXAMPLE 4

### Refolding and concentration of the renatured protein

The inclusion bodies suspension obtained at the washing stage is diluted with water so that the protein content in the suspension was 180-200 g/I. The suspension is then supplemented by glycine to a concentration of 50 mM, EDTA to a concentration of 1 mM, as well as urea or guanidine hydrochloride to a final concentration of 8M and 6M, respectively. After the dissolution of the chaotropic agent we add sodium hydroxide (NaOH) to adjust the pH value to 8.0-8.2 and then introduce β-mercaptoethanol to a final concentration of 100 mM.

The protein recovery is carried out under constant stirring for 2-6 hours, then to the refolding buffer cooled to +10-15 °C with the following composition: 20 mM glycine, 1 mM EDTA, 2.5 mM cystine, 10% glycerol, pH 11.2, a protein solution is added at a ratio of 1:100 in an amount at which the protein concentration in the refolding buffer amounts to 0.9-1.0 g/I. The protein refolding process is conducted under constant stirring for 8-12 hours. The refolding process end time is determined experimentally upon the termination of growing refolded protein concentrations according to the HPLC analysis.

Upon the protein refolding completion, zinc chloride (ZnCl₂) is added to the refolded protein solution to a final concentration of 2 mM, and then the pH is adjusted to 7.8, which corresponds to the fusion protein isopoint. Under these conditions, within 20-30 minutes a well sedimentary deposit, the spontaneous deposition of which leads to a clear separation of aqueous phases containing and not containing the protein precipitate, which allows us to decant the most (80%) of the supernatant. The fusion protein precipitate is fed to the separator (g-factor of ≥ 4000), equipped with the automatic sludge discharge. The concentrated suspension of the refolded protein sediment is collected and transferred to the enzymatic conversion stage.

### EXAMPLE 5

### Tripsinolysis of the fusion protein (cleavage by trypsin)

The refolded protein concentrated suspension obtained at the previous stage is collected in a reactor equipped with a water jacket and a stirrer. In the suspension we add water, purified to the refolded protein concentration of 10-12 mg/ml, 2M of hydrochloric acid solution to bring the pH = 2.5 - 3.0 and stir for 20-30 minutes until the protein is completely dissolved. Then we determine the refolded protein concentration in the resulting solution (2540 g by HPLC). Then we add glycine to the concentration of 100 mM. By introducing 2M of the sodium hydroxide solution we set pH within 11.0-11.5. The resulting refolded protein solution is then cooled to 10-12C. After that the fusion protein solution is loaded with 254 mg of trypsin swine (weight ratio of 10,000:1).

The tripsinolysis is performed at 10-12°- C for 16-18 hours to reach the maximum concentration of Arg-(B31)-insulin (HPLC control is carried out every 2 hours) and finished by adding a solution of 2M hydrochloric acid to a pH of 2.5 - 3.0. The 2-propanol is added to the solution of Arg-(B31)-insulin obtained in the previous stage to a concentration of 20% while stirring, then it is stored with the mixer turned off for 10-12 hours at a temperature of 10-12 ºC and fed to the centrifuge with the g-factor ≥ 14000 for the clarification. The clarified solution of the Arg-(B31)-insulin is collected in a reactor with a jacket.
The tripsinolysis results in the clarified solution containing 1650 g of Arg-(B31)-insulin (control by HPLC), which is then forwarded to the chromatographic purification stage.

### EXAMPLE 6

### Cleaning of Arg-(B31)-insulin on silicagel C8

The Arg-(B31)-insulin purification is carried out using a steel column of the dynamic axial compression (DAC) with an inner diameter of 450 mm and 1000 mm high. The column operating pressure is about 40 bar. The column is packed with the spherical silicagel, for example, Daisogel SP-120-20-C8-Bio or polymeric sorbent, for example, Amberchrom XT-20. The volume of the sorbent is about 100 liters.

The following buffer solutions are used for the purification:
composition "A": 15% 2-propanol, 0.1 % TFA;
composition "B": 50% 2-propanol, 0.05% TFA.

To carry out a purification a part of the Arg-(B31)-insulin clarified solution is used, containing from 800 to 850g of Arg-(B31)-insulin. Altogether two chromatographic purifications of the Arg-(B31)-insulin are performed.

The Arg-(B31)-insulin clarified solution obtained in the previous tripsinolysis stage is fed with the pump mounted in the liquid chromatograph at a flow rate of 3.2 I/min into the chromatographic column, which is then washed with 300 1 of buffer solution "A". The Arg-(B31)-insulin elution is carried out using a binary linear gradient to a concentration of 2-propanol from 15 to 25% within 4 hours. The sorbent regeneration is carried out in the reverse flow of the buffer solution "B". Balancing the sorbent is carried out by the buffer "A". Periodically, about once every 5-10 cycles of treatment, the sorbent is additionally regenerated in the column with a 50 mM sodium hydroxide solution in the 50%-2-propanol at the reflux.

The eluate containing the Arg-(B31)-insulin is divided into 10-15 fractions depending on the resulting volume. The fractions with over 80% of the Arg-(B31)-insulin (control by HPLC analysis) are combined in a reactor with a stirrer. After two purifications in the combined Arg-(B31)-insulin eluate we determine the Arg-(B31)-insulin content (1458g by HPLC). Then, while stirring, we add in the eluate an equal volume of purified water, citric acid to a concentration of 50 mM, phenol to a concentration of 10 mM, 10% zinc chloride solution to a concentration of 1.5 mM. By adding 2M sodium hydroxide solution we set the pH value to 7.5-8.0, and expose the composition in it for 3 to 5 minutes. Then by adding 2M hydrochloric acid solution we set the pH = 6,0-6,2 and continue stirring for the next 12 hours.

The resulting Arg-(B31)-insulin crystals are separated from the mother liquor by filtration on a pressure filter at a pressure of not more than 0.7 bar. The crystals at the pressure filter are washed with a 0.9% sodium chloride solution and discharged into the stainless steel process container to obtain 1429 g of Arg-(B31)-insulin with a purity of 86%.

### EXAMPLE 7

### Ara-(B31)-insulin ion-exchange chromatographic purification (IOC)

For the Arg-(B31)-insulin ion-exchange chromatographic purification we use a dynamic axial compression steel column (DAC) with an inner diameter of 450 mm and 1000 mm high. The column operating pressure is about 20 bar. The column is equipped with a spherical monodisperse ion-exchange sorbent, for example, Source S15, a volume of about 70 liters. The operating pressure is ≤ 20 bar.

The following buffer solutions are used for the ion-exchange chromatography:
The buffer solutions compositions:
   "A": 30% 2-propanol, 10 mM citric acid, pH 3.5;
   "B": 30% 2-propanol, 10 mM citric acid, 1.0 M NaCl, pH 3.5.

To perform the purification we use 1400 to 1450g Arg-(B31)-insulin. The Arg-(B31)-insulin crystals obtained in the previous stage are suspended in 50 I of 30% 2-propanol, to then, without stopping the stirring, set the pH 2.7-3.0 2M with the hydrochloric acid solution. The stirring is continued for 20 - 30 minutes until the Arg-(B31)-insulin completely dissolves. The resulting Arg-(B31)-insulin solution is successively filtered through a depth filter with a 0.5 micron retention ability and a membrane filter with an absolute retention of 0.2 microns.

The Arg-(B31)-insulin clarified solution obtained in the previous tripsinolysis stage is fed with the pump mounted in the liquid chromatograph at a flow rate of 3.2 I/min into the chromatographic column, which is then washed with 140 1 of buffer solution "A". The Arg-(B31)-insulin elution is carried out using a binary linear gradient to a sodium chloride concentration of 10 to 300 mM within 2,5 hours. The sorbent regeneration is carried out in the reverse flow of the buffer solution "B". Balancing the sorbent is carried out by the buffer "A". Periodically, about once every 5-10 cycles of treatment, the sorbent is additionally regenerated in the column with a 0.5 mM sodium hydroxide solution.

The eluate is divided into 10-15 fractions depending on the resulting volume. The fractions with over 90% of the Arg-(B31)-insulin (control by HPLC analysis) are combined in a reactor with a stirrer to determine the Arg-(B31)-insulin content (1330g by HPLC). Then, while stirring, we add in the eluate two volumes of purified water and carry out the Arg-(B31)-insulin crystallization, as described above in Example 6.

As a result we obtain 1302 g of Arg-(B31)-insulin with a purity of 97%.

### EXAMPLE 8

### Processing Arq-(B31)-insulin by carboxypeptidase B

The Arg-(B31)-insulin crystals obtained in the previous stage are suspended in 200 I of the treated water in a reactor equipped with a water jacket and a stirrer. Then, while stirring, we set the pH to 2.7-3.0 by adding 2M of the hydrochloric acid solution. The stirring is continued for 30 minutes until the Arg-(B31)-insulin completely dissolves. Then we determine the concentration of Arg-(B31)-insulin (1302 g by HPLC), add benzamidine hydrochloride to a concentration of 2 mM, Tris to a concentration of 25mm and 2M of sodium hydroxide to set the pH 8,0-8,5. The resulting Arg-(B31)-insulin solution is cooled to a temperature of 10-12 º C. Then, in the reactor is charged with carboxypeptidase B in an amount of 43.4 mg (the weight ratio of 30000:1). The process of enzymatic conversion is carried out at a temperature of 10-12 ºC for 18-20 hours to reach the maximum insulin concentration (control by HPLC).

Into the insulin solution obtained as a result of the enzyme conversion we add 2 M of the hydrochloric acid to set the pH at 3.0 to 3.5, while continuing to maintain the temperature within the range of 10 to 15 °C. To the insulin solution we add, while stirring, citric acid to a concentration of 50 mM, 2-propanol to a concentration of 5%, and 1.3 I of 10% zinc chloride solution.

Then with the 2M sodium hydroxide solution we set the pH value at 7.5 - 8.0 and expose the mixture in this composition for 3 to 5 minutes, then reduce the pH to 6.2 - 6.3 with 2M hydrochloric acid solution keeping on stirring for the next 2 hours. Then with 2M hydrochloric acid solution we adjust the pH to 5.8-5.9 and continue stirring for the next 6 hours. The resulting Arg-(B31)-insulin crystals are separated from the mother liquor by filtration on a pressure filter at a pressure of not more than 0.7 bar. The crystals at the pressure filter are washed with a 0.9% sodium chloride solution and discharged into the stainless steel process container to obtain 1237 g of insulin with a purity of 97%.

### EXAMPLE 9

### HPLC purification of insulin

For the HPLC purification of insulin we use a steel column of the dynamic axial compression (DAC) with an inner diameter of 450 mm and 1000 mm high. The operating pressure of the column is about 60 bar. The column is packed with a modified spherical silica gel, for example, Daisogel SP-120-10-C8-Bio (70 I). The composition used to clean the buffer solutions:
Composition "A": 10% 2-propanol, 200 mM ammonium acetate, acetic acid, 500 mM;
Composition "B": 50% 2-propanol, 100 mM ammonium acetate, acetic acid, 250 mM.

One purification requires 1200 to 1250 g of insulin. The insulin crystals obtained in the previous stage are suspended in 50 I of 10% 2-propanol, then while stirring, we add 0.75 liter of ice acetic acid and by adding 2M hydrochloric acid solution we set the pH at 3.0-3.5. The stirring is continued for 20 - 30 minutes until the insulin completely dissolves. The resulting insulin solution is filtered through a series of depth filter with 0.5 micron retention and a membrane filter with an absolute retention of 0.2 microns. Then we determine the content of insulin (1237 g by HPLC) in the resulting insulin solution.

The clarified insulin solution is fed by a liquid chromatograph pump with a flow rate of 4.0 1 / min to a chromatographic column, which is then washed with 140 liters of buffer "A". The insulin elution is carried out using a binary linear gradient to a concentration of 2-propanol from 17 to 25% within 2.5 hours. The sorbent regeneration is carried out in the reverse flow of the buffer solution "B". Balancing the sorbent is carried out with the buffer "A". Periodically (once every 15 - 20 cycles of treatment) the sorbent in the column is recovered additionally in the return flow by 50 mM of the sodium hydroxide solution in 50% of rated 2-propanol.

The eluate, containing insulin, is divided into 10 - 15 fractions. The fractions containing more than 98% insulin (control by HPLC analysis) are combined in a reactor equipped with a stirrer and a water jacket. In the resulting eluate we determine the insulin content (1175 g by HPLC). In the eluate we add, while stirring, an equal amount of purified water and 235 ml of 10% zinc chloride solution. By 2M of sodium hydroxide the pH is set to 5.4-5.5. The solution temperature is lowered to 10-15 °C and the stirring is continued for another 10-12 hours. The resulting highly purified insulin crystals are separated from the mother liquor by filtration on a pressure filter at a pressure of not more than 0.7 bar. The crystals at the pressure filter are washed with 0.9% sodium chloride and discharged into the technological stainless steel container.

The resulting crystals of highly purified insulin are fed through a transfer window into the room of the cleanliness class «D». The highly purified insulin crystals are suspended in a reactor equipped with a stirrer, in 190 1 of purified water chilled to a temperature of 10 °C, then with 2M of hydrochloric acid solution the pH is set at 3.0 to 3.5. The stirring is continued for 20-30 minutes until the complete insulin dissolution. In the insulin solution we add citric acid to a concentration of 25 mm, 2-propanol to a concentration of 5% and 600 ml of 10% zinc chloride solution. The resulting insulin solution was filtered through a depth filter with 0.5 micron retention and a sterilizing membrane filter with an absolute retention 0.2 microns.

The clarified insulin solution is collected in the prepared reactor, which is located in a room of cleanliness class "C". The room temperature is maintained at 10-15 °C. By 2M of sodium hydroxide solution the pH value is set equal to 7.5-8.0, and the mixture composition is kept at this pH for 3 to 5 minutes. Then by 2M of hydrochloric acid solution the pH is set at 6.2-6.3 and the stirring is continued for 2 hours. Then by 2M of hydrochloric acid solution the pH is set at 5.8-5.9 and the stirring is continued for the next 6 hours.

The resulting highly purified insulin crystals are separated from the mother liquor by filtration on the pressure filter (the pressure not exceeding 0.7 bar). The insulin crystals on the pressure filter are washed with the calculated amount of the injection water cooled down to 10 °C and unloaded to the trays for freeze-drying. The freeze-drying loading and unloading zone is located under the kelp in the area of the cleanliness class "B". The process of highly purified insulin crystals freeze drying is carried out for 14-16 hours in the automatic mode, whereas the crystals are frozen to a temperature of -30 º C (vacuum not less than 1 mbar), and then the temperature is gradually increased up to +15 ºC. The dried crystals of the highly purified insulin are discharged from the freeze-drying unit in a stainless steel tank and weighed.

As a result we get 1211 g on the technical mass of the insulin substance (insulin content 1151 g). The resulting insulin purity equals 99.2%, the biological activity-28.1 U/mg.

The claimed group of inventions allows us to simplify the process of the industrial production of the high purity recombinant human insulin, as well as to increase the yield of the desired product.

From the above description and the above-noted Examples, the advantages attained by the product described and obtained according to the present invention are apparent.

## Claims

1. An *E.coli* strain VL21 (DE3) (VL21 (DE3) / pMSIN4) which is deposited under deposit Accession No. SMS 7884, with International Deposit Authority Czech Collection of Microorganisms, Masaryk University, Brno, Czech Republic.

2. A recombinant human insulin produced by the *E.coli* strain VL21 (DE3) according to claim 1, or fragments and derivatives thereof.

3. The recombinant human insulin according to claim 2, for use as a medicament.

4. The recombinant human insulin according to claim 2, for use in the therapeutic treatment of diabetes.

5. Use of the recombinant human insulin according to claim 4, wherein said diabetes is type I or II diabetes.

6. A pharmaceutical composition comprising the recombinant human insulin according to claim 2 as an active ingredient and a pharmaceutically bioactive excipient.

7. A recombinant plasmid for the expression of recombinant pMSIN4 human insulin having a size of 5495 bp, wherein said recombinant plasmid consists of the following elements:
- a resistance gene to kanamycin (Kanr, 813 bp);
- a cloned gene (synthetic gene, 225 bp) encoding the insulin precursor protein, containing a prepeptide consisting of 21 amino acid residues (2.46 kDa);
- a T7 promoter (17 bp);
- an arginine codon (CGC), linking the sequences encoding the A- and B-insulin chain; and
- a replication site (ColEl origin, 588 bp).

8. The recombinant plasmid according to claim 7, wherein said resistance gene is a resistance to kanamycin (Kanr, 813 bp).

9. Use of the recombinant pMSIN4 plasmid according to claim 7, for the for the expression of recombinant human insulin.

10. A method for the production of recombinant human insulin, comprising the steps of:
a. culturing of the producer strain *Escherichia coli* according to claim 1 in a nutrient medium; and
g. purifying and obtaining the recombinant human insulin.

11. The method according to claim 10, comprising the steps of:
a. culturing of the producer strain *Escherichia coli* according to claim 1 in a nutrient medium,
b. destroying the cells by disintegration,
c. separating the inclusion bodies containing the fusion protein,
d. dissolving the inclusion bodies in the buffer with chaotropic agents,
e. allowing fusion protein refolding,
f. cleaving the protein with trypsin and/or carboxypeptidase B
g. purifying and obtaining the recombinant human insulin.


SEQUENCE LISTING

<110> WINSORT MANAGEMENT INC.

<120> Recombinant plasmid DNA pMSIN4, encoding a hybrid polypeptide - a

<130> 11469PTEP

<150> RU2011111205
<151> 2011-03-24

<160> 5

<170> BiSSAP 1.0

<210> 1
<211> 86
<212> DNA
<213> Homo sapiens

<220>
<221> source
<222> 1..86
<223> /mol_type="DNA"
      /organism="Homo sapiens"

<400> 1
aaagaagcca cgctcgccgc acactaaata cagcgcttcc accaggtggc tgccacacag     60

atgctggttc acaaagcgca tatgag                                          86


<210> 2
<211> 82
<212> DNA
<213> Homo sapiens

<220>
<221> source
<222> 1..82
<223> /mol_type="DNA"
      /organism="Homo sapiens"

<400> 2
tgcggcgagc gtggcttctt ttataccccg aaaacccgtg gcattgtgga acagtgttgc     60

accagtattt gtagcctgta tc                                              82


<210> 3
<211> 60
<212> DNA
<213> Homo sapiens

<220>
<221> source
<222> 1..60
<223> /mol_type="DNA"
      /organism="Homo sapiens"

<400> 3
cattgaattc ttagttgcag taattttcca gctgatacag gctacaaata ctggtgcaac     60


<210> 4

<211> 26
<212> DNA
<213> Homo sapiens

<220>
<221> source
<222> 1..26
<223> /mol_type="DNA"
      /organism="Homo sapiens"

<400> 4
ctcatatgcg ctttgtgaac cagcat                                          26


<210> 5
<211> 25
<212> DNA
<213> Homo sapiens

<220>
<221> source
<222> 1..25
<223> /mol_type="DNA"
      /organism="Homo sapiens"

<400> 5
cattgaattc ttagttgcag taatt                                           25